# EUROPEAN PATENT SPECIFICATION

(11) **EP 0 459 624 B1**
(45) Date of publication and mention of the grant of the patent: **24.09.1997**
(21) Application number: 91303748.7
(22) Date of filing: 25.04.1991
(51) Int. Cl.: C07K 2/00, C07K 1/00, A61K 47/42

(54) **Galactosamine-substituted poly-omega-substituted-L-glutamic and/or-aspartic acid**
Galactosamin-substituierte poly-omega-substituierte L-Glutaminsäure und/oder -Asparaginsäure
L-acide glutamique et/ou -acide aspartique galactosamine substitués poly-oméga substituées

(30) Priority: 27.04.1990 JP 113681/90
(43) Date of publication of application: 04.12.1991
(73) Proprietor: YAMANOUCHI PHARMACEUTICAL CO., LTD., Tokyo 103 (JP)
(72) Inventor: Akaike, Toshihiro, Hoya-shi, Tokyo 202 (JP); Kitada, Ichirou, Kashiwa-shi, Chiba 277 (JP); Kunou, Megumi, Koganeishi, Tokyo 184 (JP)
(74) Representative: Geering, Keith Edwin

(56) References cited:
- CHEMICAL ABSTRACTS, vol. 113, no. 2, July 09, 1990, Columbus, Ohio, USA; HAYASHI, TOSHIO et al: "Biodegradation of copoly (L-aspartic acid/L-glutamic acid) in vitro.", page 366, column 2, abstract-no. 12 070n
- CHEMICAL ABSTRACTS, vol. 113, no. 2, July 09, 1990, Columbus, Ohio, USA; YOKOYAMA, MASAYUKI et al, page 364, column 1, abstract -no. 12 043f & J. Controlled Release 1990, 11(1-3), 269-78
- CHEMICAL ABSTRACTS, vol. 112, no. 22, May 28, 1990, Columbus, Ohio, USA; IWATSUKI, MAKOTO et al: "Biodegradable sustained-release materials containing aspartic acid polymers", page 405, column 1, abstract-no. 204 790y
- CHEMICAL ABSTRACTS, vol. 112, no. 10, March 05, 1990, Columbus, Ohio, USA; GIAMMONA, GAETANO et al: "Polymeric prodrugs: alpha, beta-poly(N-hydroxyethyl)-D,L-aspartamide as a macro- molecular carrier for some nonsteroidal antiinflammatory agents", page 437, column 1, abstract-no. 84 066t
- CHEMICAL ABSTRACTS, vol. 105, no. 26, December 29, 1986, Columbus, Ohio, USA; ZUNINO, F. et al: "Poly(L-aspartic acid) as a carrier of doxorubicin: antitumor effects on murine solid tumors", page 359, column 1, abstract-no. 232 384t

## Description

The present invention relates to galactosamine-substituted poly-ω-substituted-L-glutamic and/or -aspartic acids which are useful as macro-molecular materials for medical use, especially as missile drug carriers.

In glycoprotein in serum, a sugar structure called sialic acid-galactose-N-acetylglucosamine is omnipresent at the termini thereof. In the late 1960's, G.Ashwell and A.Morell clarified that this triose structure was required for serum protein to be stably present in blood. When sialic acid present at the termini is eliminated, galactose becomes a new sugar end. Glycoprotein from which sialic acid has been removed so that galactose is exposed is called asialo glycoprotein. Asialo glycoprotein cannot be stably present in blood flow and rapidly disappears from blood flow. It is revealed that more than about 80% of the disappeared asialo glycoprotein is taken up into liver.

Specific sugar recognition receptors are present on the surface of hepatocyte membranes. Asialo glycoprotein is taken up into cells via this asialo glycoprotein receptor. We have made investigations, paying attention to this asialo glycoprotein receptor on hepatocyte membrane and aiming at developing macro-molecular materials for drug carriers used in missile drugs, etc. As a result, it has been found that polyamino acids in which galactosamine has been introduced as a sugar residue have excellent properties.

The present invention provides galactosamine-substituted poly-ω-alkyl (and/or benzyl)-L-glutamic and/or -aspartic acids of formula : 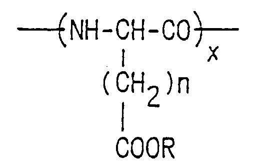 in which part or all of the constituent peptide of said polypeptide is replaced by ω-galactosamyl-L-glutamic and/or -aspartic acid residue(s) of formula : 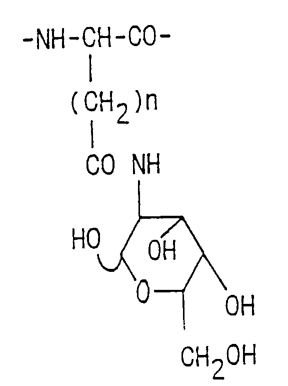 and optionally also by L-glutamic and/or -aspartic acid residue(s) of formula : 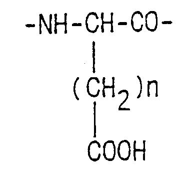 wherein X represents a polymerisation degree of 60 to 250, each n is selected independently from 1 and 2; and each R is selected independently from lower alkyl (C₁-C₆) and benzyl groups.

Preferred polypeptide of the present invention is as follows :
Structural Units :
   ω-alkyl (and/or benzyl)-L-glutamic and/or -aspartic acid residue(s) : 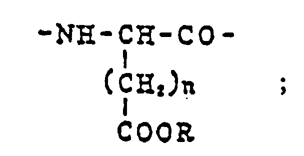
L-glutamic and/or -aspartic acid residue(s) : 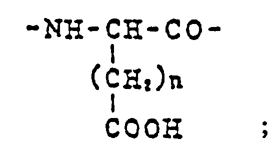
and ω-galactosamyl-L-glutamic and/or -aspartic acid residue(s) : 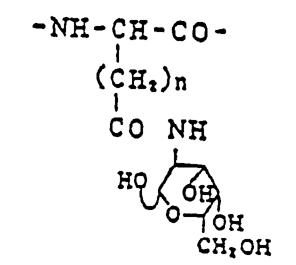 wherein n is as defined above.
   - State of configuration :: linear
   - Molecular weight :: 8,000 to 71,000
   - Polymerisation degree :: 60 to 250
Ratio of the constituent units (molar) :
   ω-alkyl (and/or benzyl)-L-glutamic and/or -aspartic acid residue(s) :
   0 - 97%
L-glutamic and/or -aspartic acid residue(s):
   0 - 87%
ω-galactosamyl-L-glutamic and/or -aspartic acid residue(s) :
   3 - 100%

The compounds of the present invention can be synthesized by, for example, the following process : 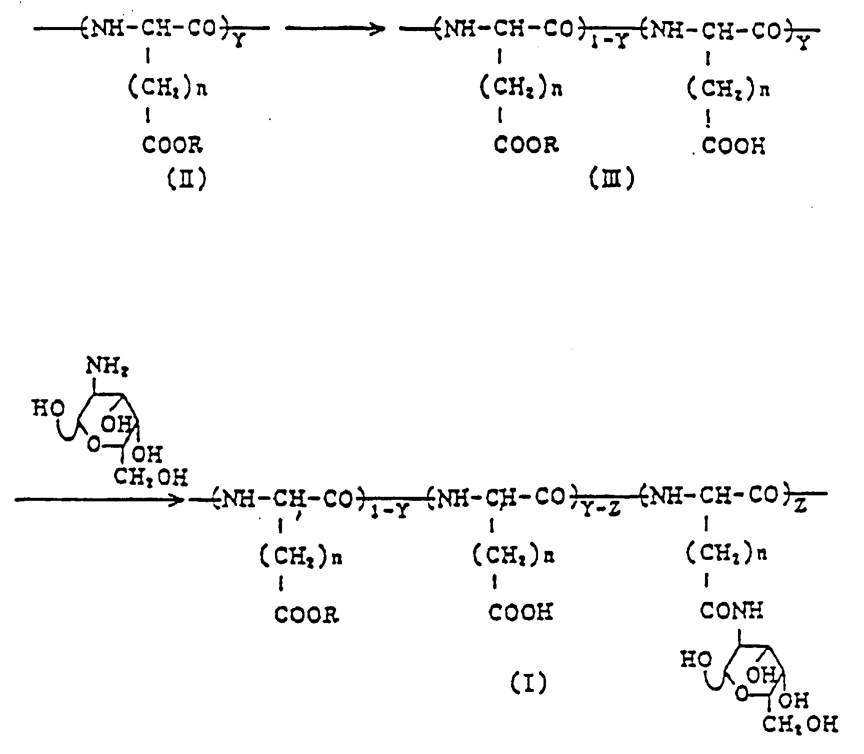 wherein n and R are as defined above, Y and Z are each less than 1, and Y ≧ Z.

The process can be carried out by hydrolyzing the ester side chain of polymer (II) to obtain in polymer (III) having free carboxyl group side chains (first step), and then introducing galactosamine into such side chain carboxyl groups to obtain desired compound (I) of the present invention (second step).

Hydrolysis in the first step can be readily carried out by treating poly-γ-alkyl (and/or benzyl)-L-glutamic and/or -L-aspartic acid with base in appropriate organic solvent. As the organic solvent, halogenated hydrocarbon (helix solvent) such as chloroform, dichloromethane, etc. are preferred but random coil solvents such as dichloroacetic acid, trifluoroacetic acid, etc. may also be used.

As the base, sodium hydroxide, potassium hydroxide, etc. are appropriate. Such base can be added to the reaction solution as solution in aqueous alcohol (such as methanol, isopropyl alcohol, etc ).

The reaction is suitably carried out at about room temperature for 10 to 200 minutes.

By appropriately choosing the reaction conditions, especially reacton time, the extent of the hydrolysis may be regulated.

As the starting compounds (II) for this step, compounds having a polymerisation degree of about 60 to 250 are used. In the examples later described, for example, poly-γ -methyl-L-glutamate (simply referred to as PMLG) having a polymerisation degree of approximately 100 to 200 (molecular weight of about 14,000 - 29,000) was used.

The second step is peptization between the side chain carboxyl groups of polymer (III) and the primary amino group of galactosamine. For this one may activate said carboxyl or amino, group, or operate in the presence of condensing agent.

The carboxyl group of hydrolysate (III) may for example be activated by conversion to the p-nitrophenyl ester. After the activated compound is isolated, galactosamine is reacted with it, suitably in a solvent such as dimethylformide (DMF), tetrahydrofuran (THF), dimethylsulfoxide (DMSO), etc., , at room temperature or under cooling. The reaction period is several hours to several days. The rate at which peptization proceeds may be determined by quantitative assay of isolated p-nitrophenol associated with the reaction.

The process using a condensing agent comprises coupling the partial hydrolysate (III) with galactosamine in the presence of, e.g., N,N'-dicyclohexylcarbodiimide (DCC) , 1-ethyl-3-(3-dimethylaminopropyl) carbodiimide hydrochloride (EDC), etc. The reaction conditions can be as for the aforesaid peptization of an activated carboxyl group.

The resulting desired product (I) can be purified by dialysis using, e.g. cellulose dialysis membrane.

The compounds of the present invention can be applied in the medical field as macro-molecular compounds for recognising vital cells. They are vitally degradative and water-soluble since they are polyamino acid derivatives which are macro-molecular materials similar to natural macro-molecular materials. Accordingly, the compounds are preferred as macro-molecular materials for drug carriers used as missile drugs, etc.

The affinity of compounds according to the present invention for hepatocytes is shown by animal tests using rats.

### Experiment

Using SD strain female rats (age of 4 to 5 weeks), rat hepatocytes were isolated by modification of so-called Seglen's perfusion method of digesting intercellular adhesive protein with enzyme. The prepared hepatocytes were suspended in ice-cold WE medium (400,000 cells/ml). Then, 1.5 ml of the hepatocyte suspension was inoculated on each polymer-coated Petri dish using a disposable pipette followed by culturing at 37°C in a carbon dioxide gas concentration of 5% for a predetermined period in a carbon dioxide gas culture device. Non-adhesive cells were then counted to determine the rate of adhesion.

The polymers used in this experiment are a compound of the present invention (poly-γ-methyl-L-glutamate, abbreviated as PGA) and polyvinyl type polymer (polyvinyl-benzyllactonamide, abbreviated as PVLA) which is conventionally known to have affinity to hepatocytes.

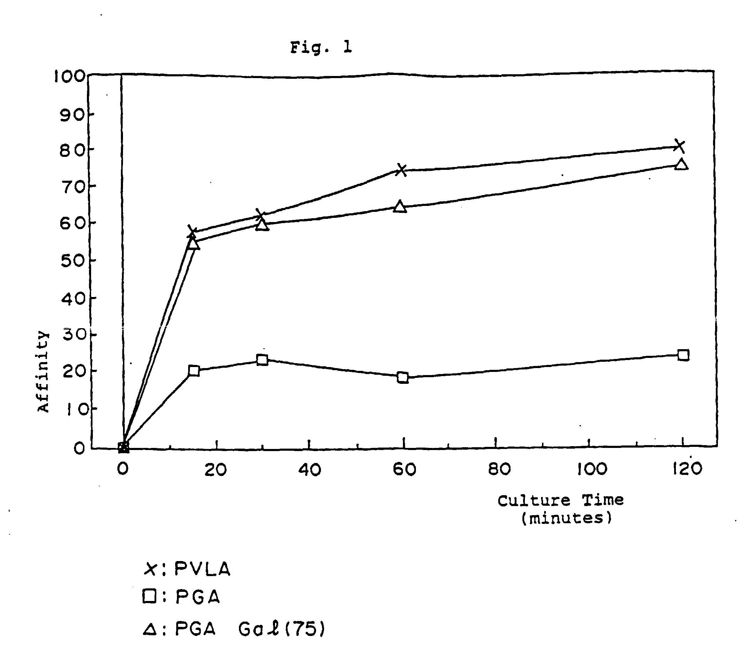

The adhesion to hepatocytes in each Petri dish is shown in Fig.1. The graphs show that hepatocytes have little adhesion to the main chain polymer and there is no physiological activity on the main chain itself, whereas the polymer of the present invention having galactosamine on the side chain thereof shows a high rate of adhesion as in PVLA.

### Preparation of polymer-coated Petri dish :

Each sample was dissolved in milli Q water at a concentration of 0.05% (W/V). In a Petri dish 2 ml of the polymer solution was injected followed by freeze drying. Subsequently by rinsing with milli Q water 3 times and drying naturally, a polymer-coated Petri dish was prepared.

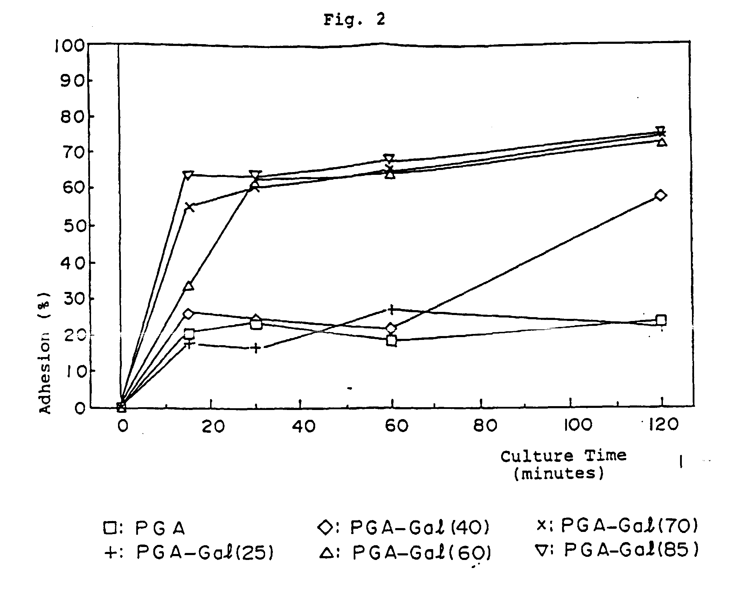

Fig.2 shows adhesion to polymer-coated Petri dishes of compounds of the present invention having different degrees of galactosamine substitution. The graphs show that hepatocytes adhere little to the main chaim polymer and to the polymer having a sugar content of 25%. For the polymers having higher sugar contents of 40%, 60%, 70% and 85%, an increased adhesion to hepatic cells was noted. Samples having sugar residue contents of 60% or more showed almost the same adhesion behaviour.

There are various pharmaceutical forms for the compounds of the present invention. Below is shown one example for a nanosphere preparation.

Lipiodol, iso-butyl cyanoacrylate and a medicinal compound were dissolved in ethanol. Non-ionic surfactant and a compound of the present invention were dissolved in water, and to the resultant aqueous solution was added the above ethanol solution under vigorous stirring. After lyophilization, a nanosphere preparation containing the compound of the present invention and the medicament was obtained. (cf. Ref. Int. J.Pharm. 86, 125-132 (1986)).

The compounds of the present invention and their preparation are illustrated by the following Examples.

### Example 1

### (1) Hydrolysis of side chain methyl ester of PMLG

In 100 ml of chloroform was dissolved 11.57 of PMLG to give an 8% solution. While stirring, a mixture of 35.8 ml of 2N-sodium hydroxide, 71.5 ml of methanol and 71.5 ml of isopropyl alcohol (volume ratio 1 : 2 : 2 ) was added dropwise to the solution over 15 minutes. Sturring was then continued at room temperature, whereby hydrolysis of the side chain methyl ester was carried out; extent of hydrolysis was varied by varying the stirring time. Then the reaction mixture was neutralised with glacial acetic acid to terminate the reaction. While stirring, the reaction solution was added to 500 ml of diethyl ether to precipitate the product. The precipitates were then filtered. After washing with diethyl ether several times, a small amount of distilled water was added to the precipitates and the resulting gel was packed in a dialysis tube. Dialysis was performed at room temperature for 2 days. By subsequent freeze drying, the side chain-hydrolysed polymer was prepared. The dialysate was appropriately exchanged.

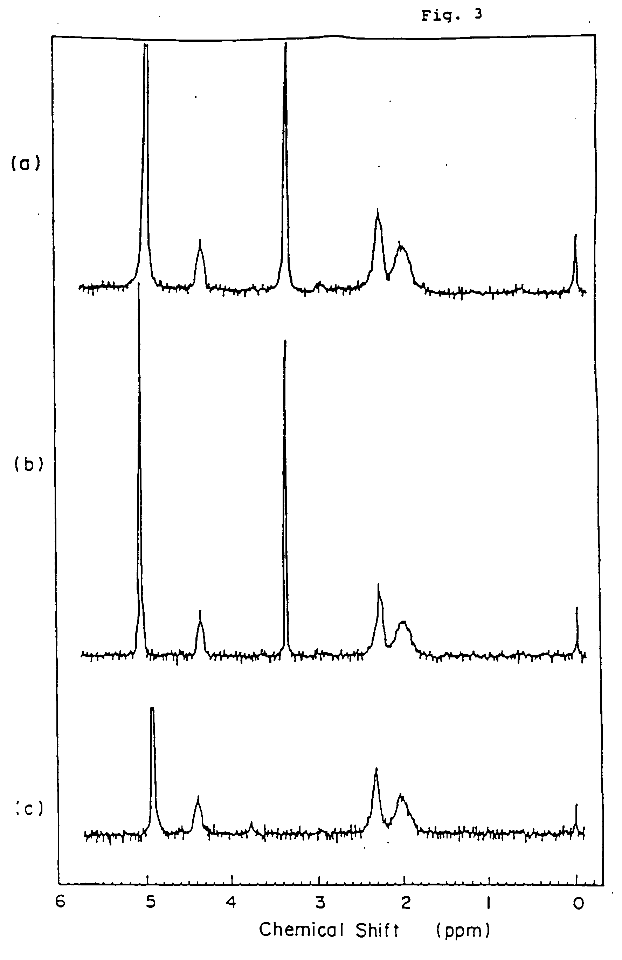

¹H-NMR spectra of the resulting side chain-hydrolysed polymers are shown in Fig.3. In the Figure, spectra (a), (b) and (c) are for hydrolysates resulting from different reaction times increasing from (a) to (c); spectrum (c) was obtained after the longest reaction - at room temperature for 3 days. The results reveal that the peak of the side chain methyl ester decreases from (a) to (c), indicating that the extent of side chain hydrolysis increases with reaction time.

### (2) Activation of the side chain carboxyl group

After 0.8 g (5.9 x 10⁻³ mol, value calculated from apparent molecular weight per 1 monomer unit) of the partially hydrolysed polymer and 0.55 g (4.0 x 10⁻³ mol) of p-nitrophenol were added to 20 ml of DMF, 0.82 g (4.0 x 10⁻³ mol) of DCC was added to the solution. The reaction was carried out by stirring at 0°C for 30 minutes and then at room temperature for 2 days. Thereafter, the mixture was allowed to stand for 2 hours in a refrigerator. After thoroughly washing with DMF, water and hot ethanol in this order, the precipitates were dried in vacuum to prepare a sample. This method is for polymer having a degree of side chain in ester hydrolysis lysis of 28.6%; in other reactions, amounts of p-nitrophenol and DCC were made 1.5 to 2 times the molarity of the carboxyl group in the polymer side chain.

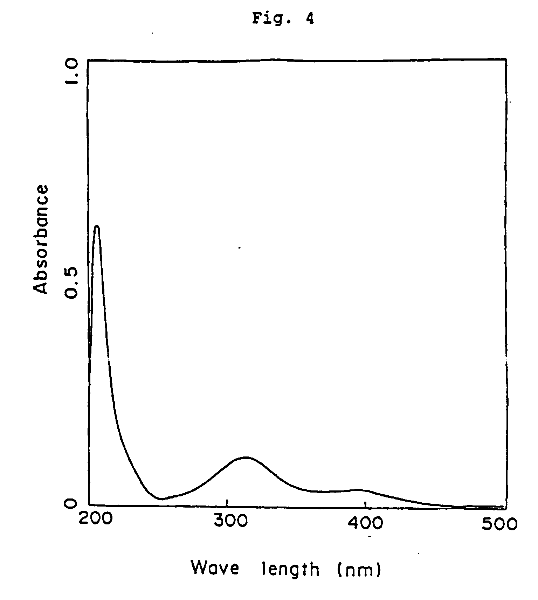

UV spectrum of the obtained compound is shown in Fig.4. In the figure, the peak of p-nitrophenol is observed at 310 nm, confirming that p-nitrophenyl was introduced into the polymer side chain.

The extent of side chain activation (p-nitrophenyl introduction) was measured by UV spectroscopy, using a method which comprises dissolving the reaction product in methanol to a concentration of 0.2 g/l, adding 0.1 N potassium hydroxide to the solution, vigorously stirring the mixture for 10 minutes and measuring the absorption of p-nitrophenyl in the solution appearing at 390 nm.

### (3) Coupling with galactosamine (activated ester method)

In 10 ml of DMF was dissolved 0.22 g of galactosamine hydrochloride (1.04 x 10⁻³ mol). After 0.15 ml (1.04 x 10⁻³ mol) of triethylamine was added to the solution, 0.30 g (1.84 x 10⁻³ mol, value calculated from apparatent molecular weight per 1 unit) of the activated polymer was added to the mixture. The reaction was carried out at room temperature for 2 days. Then the solution containing the precipitates was dialysed (2 days) and then freeze dried to prepare a sample. The amounts given here are for the above sample obtained by activation of the side chain using the polymer having a side chain hydrolydsis extent of 28.6%; for other samples, about two-fold amounts of sugar and triethylamine were used relative to the rate of activation of the side chain.

### (Method using condensing agent)

After 0.45 aliquots of PGA were dissolved in water, galactosamine (Gal - NH₂) was dissolved therein in respective 1.5, 1, 0.75, 0.5 and 0.25-fold mol amounts based on the side chain carboxyl group. The pH of the solution was adjusted to 4.7 with 0.1 N hydrochloric acid. An aqueous solution having pH of 4.7, in which EDC was dissolved in a 1.5-fold mol amount based on the galactosamine used in the solution, was added dropwise to the solution at 0°C over 8 hours. Subsequently, the reaction was carried out at room temperature for 24 hours and then dialysed for 2 days. By freeze drying, samples were prepared.

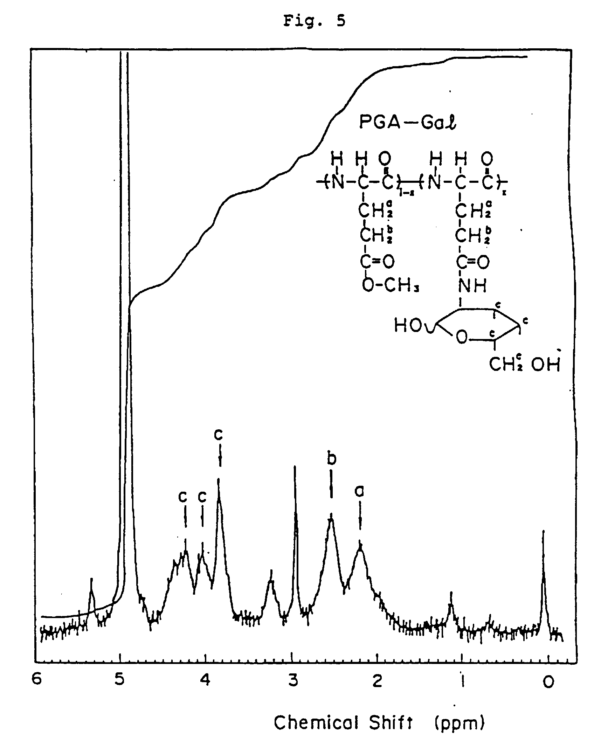

The measurement results of ¹H-NMR spectra of the resulting compounds (PGA-Gal) obtained in these reactions are shown in Fig.5. As is clear from the Figure, the peak of the sugar was observed at about 4 ppm in each sample, confirming that the sugar was introduced into the polymer side chain.

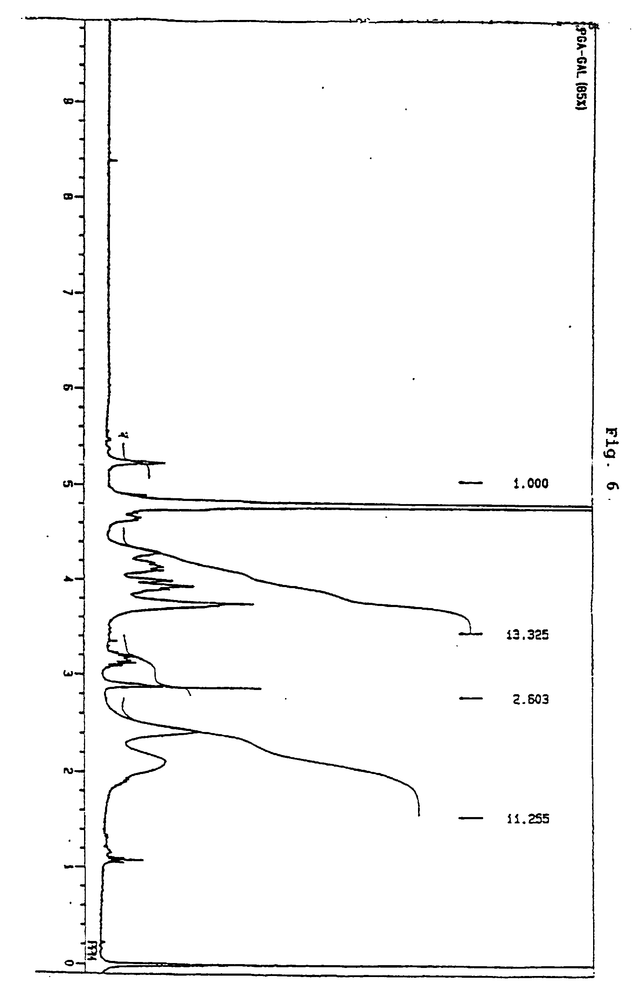

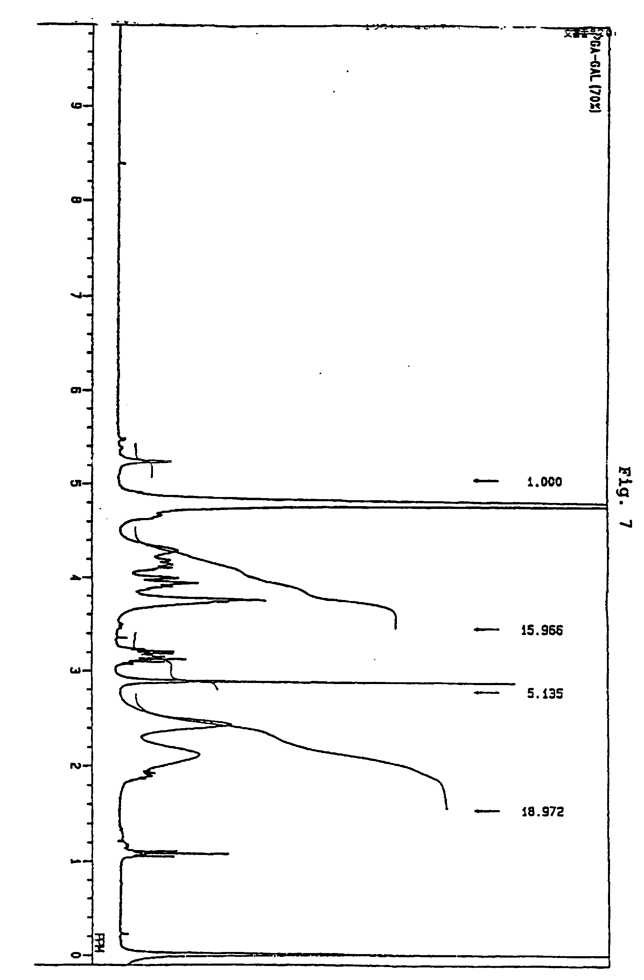

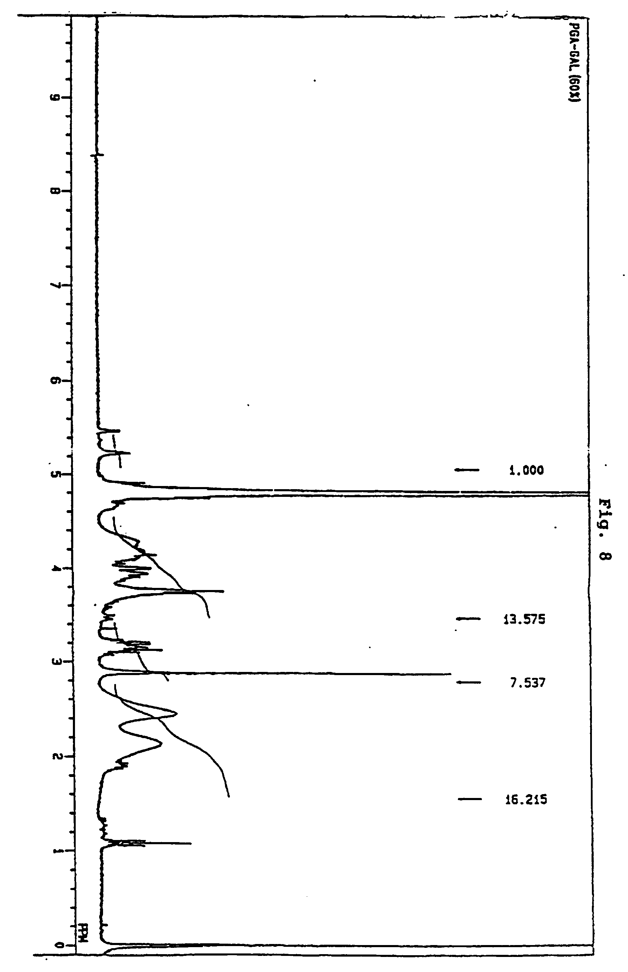

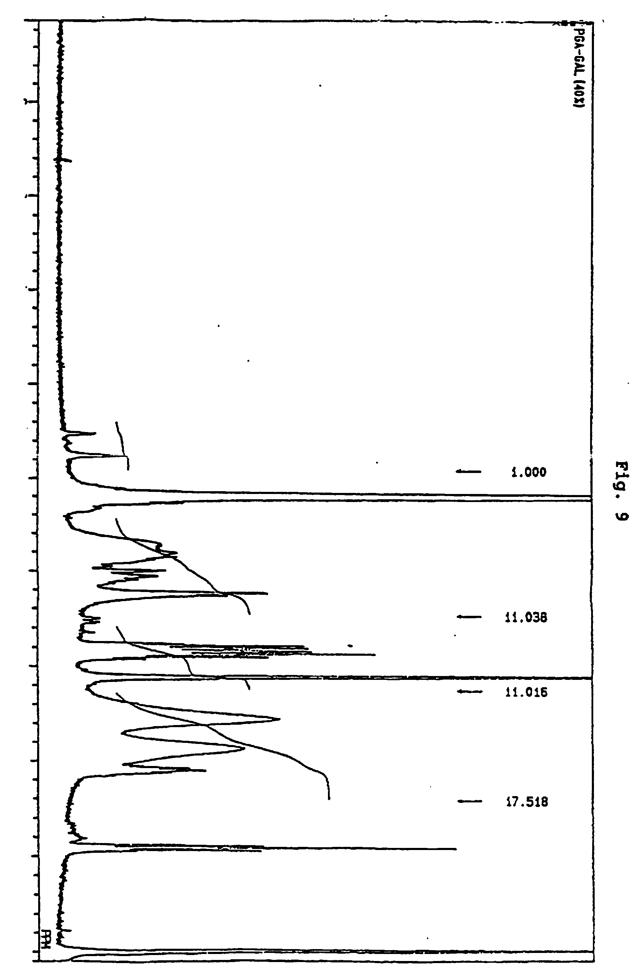

The measurement results of ¹H-NMR spectra of the resulting galactosamine substituted compounds (PGA-Gal) obtained in the above condensation reaction (for galactosamine used in 1.5, 1, 0.75 and 0.5 mol amounts based on the side chain carboxyl group) are shown in Fig.6, Fig.7, Fig.8 and Fig.9 respectively. As is clear from the Figures, the PGA-Gal compouund-85 (galactosamine substitution rate 85%) the PGA-Gal compound 70 (galactosamine substitution rate 70%), the PGA-Gal compound-60 (galactosamine substitution rate 60%) and the PGA-Gal compopund-40 (galactosamine substitution rate 40%) were obtained according the the amount of galactosamine used.

### Brief Description of the Drawings

FIGURE 1 shows affinity of compound of the present invention (PGA-Gal : substitution rate 75%), starting compound (PGA) and control (PVLA) for rat hepatocytes.

FIGURE 2 shows the difference in adhesion to hepatic cells of compounds of the present inven tion having different sugar contents.

FIGURE 3 shows ¹H-NMR spectra indicating the progress of hydrolysis of PMLG at the side chain methyl ester. In the Figure, (c) is for the product obtained after reacting at room temperature for 3 days.

FIGURE 4 is the UV spectrum of the compound obtained in Example 1 (2).

FIGURE 5 is the ¹H-NMR spectrum of the compound obtained in Example 1 (3).

FIGURE 6 is the ¹H-NMR spectrum of the PGA-Gal compound-85.

FIGURE 7 is the ¹H-NMR spectrum of the PGA-Gal compound-70.

FIGURE 8 is the ¹H-NMR spectrum of the PGA-Gal compound-60.

FIGURE 9 is the ¹H-NMR spectrum of the PGA-Gal compound-40.

## Claims

1. A galactosamine-substituted poly- ω -alkyl (and/or benzyl)-L-glutamic and/or -aspartic acid of formula : 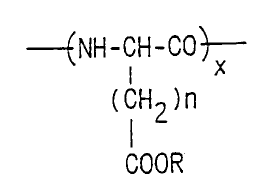 in which part or all of the constituent peptide of said polypeptide is replaced by ω-galactosamyl-L-glutamic and/or - aspartic acid residue(s) of formula : 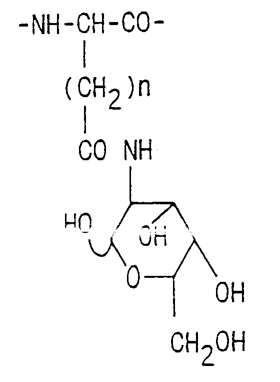 wherein X represents a polymerisation degree of 60 to 250; each n is selected independently from 1 and 2; and each R is selected independently from C₁-C₆ alkyl and benzyl groups.

2. A galactosamylated ω-alkyl (and/or benzyl)-L-glutamic and/or -aspartic acid linear polymer whose constituent units are selected from
(1) ω-alkyl (and/or benzyl)-L-glutamic and/or -aspartic acid residue(s) 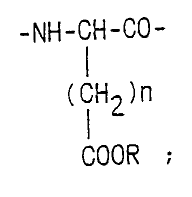
(2) L-glutamic and/or -aspartic acid residue(s) 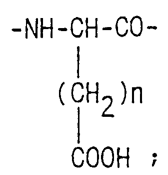 and
(3) ω-galactosamyl-L-glutamic and/or -aspartic acid residue(s) 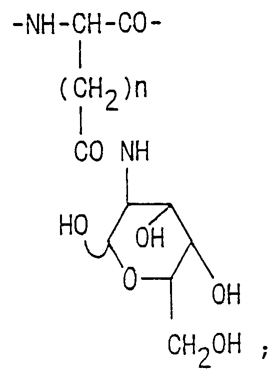
wherein each n is selected independently from 1 and 2 and each R is selected independently from C₁-C₆ alkyl and benzyl groups, said polymer having a polymerisation degree of 60 to 250 and a molecular weight of 8,000 to 71,000 and containing (molar basis)
0 - 97% of (1),
0 - 87% of (2) and
3 - 100% of (3).

3. A method of preparing galactosamine-substituted poly-ω-C₁-C₆ alkyl (and/or benzyl)-L-glutamic and/or -aspartic acid which comprises hydrolysing to carboxyl side chains at least some of the ester side chains of poly- ω -C₁-C₆ alkyl (and/or benzyl)-L-glutamic and/or -aspartic acid and peptizing at least some of the resulting carboxyl side chains with galactosamine, optionally after activation of reactant carboxyl and/or amino groups.

## Patentansprüche

1. Galactosamin-substituierte Poly-ω-alkyl- (und/oder -benzyl)-L-glutamin- und/oder -asparaginsäure der Formel 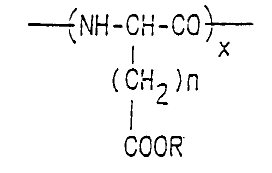 wobei ein Teil oder die Gesamtheit des einen Bestandteil des Polypeptids bildenden Peptids ersetzt ist durch ω-Galactosamyl-L-glutamin- und/oder -asparaginsäurerest(e) der Formel: 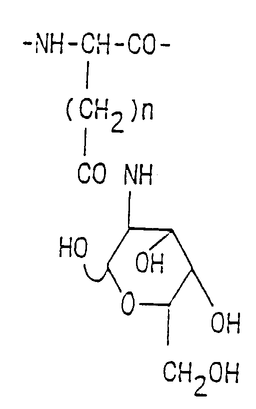 wobei X einen Polymerisationsgrad von 60 bis 250 angibt, jedes n unabhängig ausgewählt ist aus 1 und 2 und jedes R unabhängig ausgewählt ist aus C₁-C₆-Alkyl- und Benzylgruppen.

2. Lineares galactosamyliertes ω-Alkyl- (und/oder -Benzyl)-L-glutamin- und/oder -asparaginsäurepolymer, dessen Grundeinheiten ausgewählt sind aus
(1) ω-Alkyl- (und/oder -Benzyl)-L-qlutamin- und/oder -asparaginsäurerest(en) 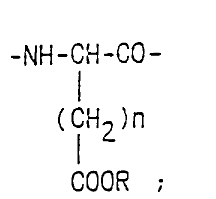
(2) L-Glutamin- und/oder -asparaginsäurerest(en) 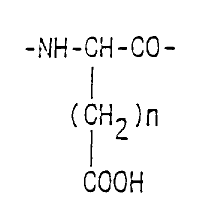 und
(3) ω-Galactosamyl-L-glutamin- und/oder -asparaginsäurerest(en) 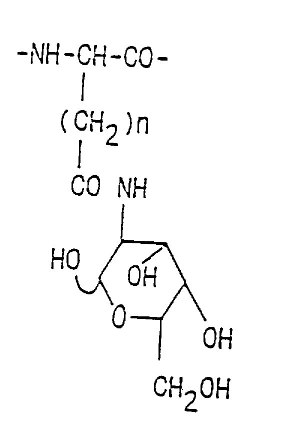 wobei jedes n unabhängig ausgewählt ist aus 1 und 2 und jedes R unabhängig ausgewählt ist aus C₁-C₆-Alkyl- und Benzylgruppen, wobei das Polymer einen Polymerisationsgrad von 60 bis 250 aufweist und ein Molekulargewicht von 8000 bis 71000 und enthaltend (molare Basis)
0 - 97 % (1),
0 - 87 % (2) und
3 - 100 % (3).

3. Verfahren zur Herstellung von Galactosamin-substituierter Poly-ω-C₁-C₆-alkyl- (und/oder -benzyl)-L-glutamin- und/oder -asparaginsäure, umfassend das Hydrolysieren mindestens einiger der Esterseitenketten von Poly-ω-C₁-C₆-alkyl- (und/oder -benzyl)-L-glutamin- und/oder -asparaginsäure zu Carboxylseitenketten und Peptisieren mindestens einiger der entstandenen Carboxylseitenketten mit Galactosamin, gegebenenfalls nach Aktivierunq der Carboxyl- und/oder Aminogruppen des Reaktionspartners.

## Revendications

1. Un acide galactosamine substitué poly-ω-alkyl (et/ou benzyl) L-glutamique et/ou-aspartique de formule 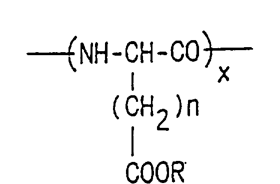 dans laquelle une partie ou tous les constituants peptide dudit polypeptide sont remplacés par un(des) résidu(s) d'acide ω-galactosamyl-L-glutamique et/ou -aspartique de formule : 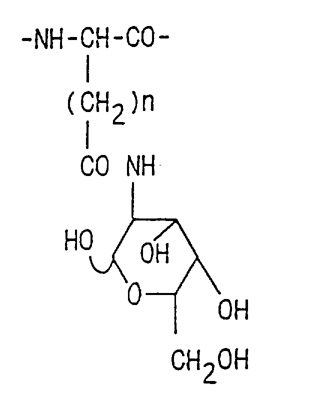 dans laquelle X représente un degré de polymérisation de 60 à 250 ; chaque n est choisi de manière indépendante parmi 1 et 2 ; et chaque R est choisi de manière indépendante parmi des groupes alkyle en C₁-C₆ et benzyle.

2. Un polymère linéaire d'acide ω-alkyl (et/ou benzyl) L-glutamique et/ou -aspartique galactoamylé dont les unités constitutives sont choisies à partir
(1) d'un (de) résidu(s) d'acide ω-alkyl (et/ou benzyl)-L-glutamique et/ou -aspartique 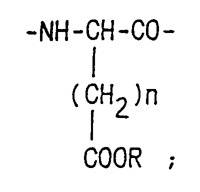 (2) d'un (de) résidu(s) d'acide L-glutamique et/ou -aspartique 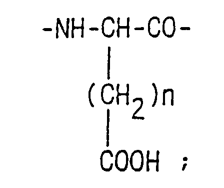 et
(3) d'un (de) résidu(s) d'acide ω-galactosamyl-L-glutamique et/ou -aspartique 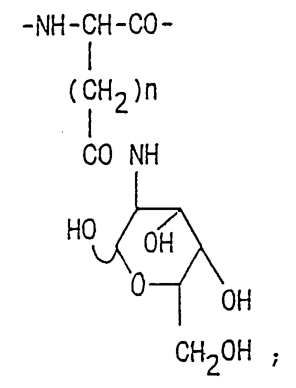 dans lesquels chaque n est choisi de manière indépendante parmi 1 et 2 et chaque R est choisi de manière indépendante parmi des groupes alkyle en C₁-C₆ et benzyle, ledit polymère ayant un degré de polymérisation de 60 à 250 et un poids moléculaire de 8000 à 71000 et contenant (base molaire)
0 - 97 % de (1)
0 - 87 % de (2) et
3 - 100 % de (3).

3. Un procédé de préparation d'acide galactosamine substitué poly-ω-alkyl en C₁-C₆ (et/ou benzyl)-L-glutamique et/ou -aspartique qui comprend l'hydrolyse des chaînes latérales carboxyle d'au moins certaines des chaînes latérales d'ester d'acide poly-ω-alkyl en C₁-C₆ (et/ou benzyl)-L-glutamique et/ou -aspartique et la peptisation d'au moins certaines des chaînes latérales carboxyle résultantes avec de la galactosamine, optionnellement après activation des groupes carboxyle et/ou amino réactifs.
